# EUROPEAN PATENT APPLICATION

(11) **EP 4 246 128 A1**
(43) Date of publication of application: **20.09.2023**
(21) Application number: 23161783.8
(22) Date of filing: 14.03.2023
(51) Int. Cl.: G01N 21/359, G01N 21/3563, G01N 21/85, G01N 33/10, G01N 21/27, G01N 21/47, G01N 21/84, G01N 21/03

(54) **GRANULAR-SUBSTANCE COMPONENT MEASURING METHOD AND GRANULAR-SUBSTANCE COMPONENT MEASURING INSTRUMENT**

(30) Priority: 18.03.2022 JP 2022044314
(71) Applicant: Topcon Corporation, Tokyo 174-8580 (JP)
(72) Inventor: YAMAUCHI, Shun, Tokyo-to, 174-8580 (JP); HAIJIMA, Yasuhito, Tokyo-to, 174-8580 (JP)
(74) Representative: Louis Pöhlau Lohrentz

(57) **Abstract**

A granular-substance component measuring method comprises steps of emitting detection light to granular substances filled in a space, receiving a reflected light from the granular substances and measuring a component of granular substances by a spectroscopy, and a displacement is given to the granular substances at each measurement, measurements are performed a plurality of times, and measurement results are averaged.

## Description

### BACKGROUND OF THE INVENTION

The present invention relates to a granular-substance component measuring method and a granular-substance component measuring instrument which measure components contained in a granular substance such as grain by a spectroscopic method.

With grain as object to be measured, analyzing instruments which optically analyze components contained in grain are known.

By analyzing components contained in grain and by measuring content rates of components such as protein/moisture content rates based on the analysis result, it is possible to measure a quality of grain as objects.

It is important to measure grain qualities and to hold predetermined qualities in supplying grain with stable qualities.

Conventionally, in a case where grain is spectroscopically analyzed, analysis samples are sampled from harvested grain as appropriate, sampled samples are filled in a predetermined container, this container is set on an analyzing instrument, and component analysis of a sample in the container is performed.

Therefore, procedures from sampling of the samples to setting on an analyzing instrument and moreover, replacement of samples and the like are complicated, and there is a problem that a time required for analyzing one sample becomes longer.

Further, there is a possibility that a difference of state of object influences a measurement result and variations occurs in a measurement result.

### SUMMARY OF THE INVENTION

It is an object of the present invention to provide a granular-substance component measuring method which improves a component measurement accuracy and a granular-substance component measuring instrument which reduces a time required for a sample analysis and improves an accuracy as an analysis data and a reliability.

To attain the object as described above, a granular-substance component measuring method according to the present embodiment comprises steps of emitting detection light to granular substances filled in a space, receiving a reflected light from the granular substances and measuring a component of granular substances by a spectroscopy, and a displacement is given to the granular substances at each measurement, measurements are performed a plurality of times, and measurement results are averaged.

Further, in the granular-substance component measuring method according to a preferred embodiment, a displacement is intermittently given to the granular substances, and a measurement is performed while the granular substances are in a stationary state.

Further, in the granular-substance component measuring method according to a preferred embodiment, the granular substances are fluidized or agitated, and a measurement is performed in a state where a dynamic displacement is given to the granular substances.

Further, a granular-substance component measuring instrument according to the present embodiment comprises a sample holding device with a space in which granular substances are filled and at least one component analyzing instrument mounted on the sample holding device, and a detection window is provided in a board facing the space, and the component analyzing instrument is configured to irradiate a detection light to the granular substances through the detection window, to receive a reflected light from the granular substances, and to measure components by a spectroscopic analysis, and the sample holding device has a displacement giving means for giving a displacement to the granular substances, and is configured to give a displacement the granular substances by the displacement giving means, and the component analyzing instrument is configured to perform measuring granular substances a plurality of times and a displacement is given to the granular substances at each measurement and to average measurement results.

Further, in the granular-substance component measuring instrument according to a preferred embodiment, detection windows are provided in two opposing boards facing the space respectively, component analyzing instruments are provided at the detection windows respectively, and components of the granular substances are measured through the detection windows by respective the component analyzing instruments.

Further, in the granular-substance component measuring instrument according to a preferred embodiment, one of the component analyzing instruments is a reference-component analyzing instrument, and a measurement result of the other component analyzing instrument is calibrated by a measurement result of the reference-component analyzing instrument.

Further, in the granular-substance component measuring instrument according to a preferred embodiment, the two component analyzing instruments are component analyzing instruments of a same type or different types.

Further, in the granular-substance component measuring instrument according to a preferred embodiment, an upper-part opening/closing valve is provided on an upper end of the space, a lower-part opening/closing valve is provided on a lower end of the space, and a displacement is given to granular substances inside the space at least by an opening/closing of a lower-part opening/closing valve.

Further, in the granular-substance component measuring instrument according to a preferred embodiment, at least the lower-part opening/closing valve is opened by a predetermined opening degree, the granular substances are discharged, and granular substances in the space are adapted to flow.

Furthermore, in the granular-substance component measuring instrument according to a preferred embodiment, an air is ejected from a lower end part of the space, and the granular substances are adapted to float/move or to be agitated.

According to the present embodiment, a granular-substance component measuring method comprises steps of emitting detection light to granular substances filled in a space, receiving a reflected light from the granular substances and measuring a component of granular substances by a spectroscopy, and a displacement is given to the granular substances at each measurement, measurements are performed a plurality of times, and measurement results are averaged. As a result, it is possible to exclude an influence which a difference of state of granular substance exerts on a measurement result and to improve a reliability of an accuracy of a measurement result.

Further, according to the present embodiment, a granular-substance component measuring instrument comprises a sample holding device with a space in which granular substances are filled and at least one component analyzing instrument mounted on the sample holding device, and a detection window is provided in a board facing the space, and the component analyzing instrument is configured to irradiate a detection light to the granular substances through the detection window, to receive a reflected light from the granular substances, and to measure components by a spectroscopic analysis, and the sample holding device has a displacement giving means for giving a displacement to the granular substances, and is configured to give a displacement the granular substances by the displacement giving means, and the component analyzing instrument is configured to perform measuring granular substances a plurality of times and a displacement is given to the granular substances at each measurement and to average measurement results. As a result, only filling a granular substance in the space and discharging a granular substance from the space, it is possible to easily set and replace a granular substance (sample), to exclude an influence which a difference of a state of a granular substance exerts on measurement results, and to improve a reliability of an accuracy of a measurement result.

### BRIEF DESCRIPTION OF THE DRAWINGS

FIG.1 is a perspective view of a granular-substance component measuring instrument according to an embodiment of the present invention.
FIG.2 is a cross-sectional view of a sample holding device according to the present embodiment.
FIG.3 is a graph to show a comparison in a spectroscopic data between flowing wheat and stationary wheat.
FIG.4 is a perspective view of the sample holding device.
FIG.5 is an arrow view along line A in FIG.4.
FIG.6 is a perspective view of the sample holding device.
FIG.7 is a perspective view of the sample holding device.
FIG.8 is a schematic diagram of a first component analyzing instrument using a white LED as a light emission source.
FIG.9 is a graph to show optical characteristics (wavelength transmission characteristics) of an optical filter of the present embodiment.
FIG.10 is a graph to show a dimming action of the optical filter.
FIG.11 is a drawing to show a reflection spectroscopy data at a calibration and a reflection spectroscopy data at an actual measurement.
FIG.12 is a schematical diagram of a second component analyzing instrument using a halogen lamp as a light emission source.

### DETAILED DESCRIPTION OF THE PREFERRED EMBODIMENTS

A description will be given below on the present embodiment by referring to the attached drawings.

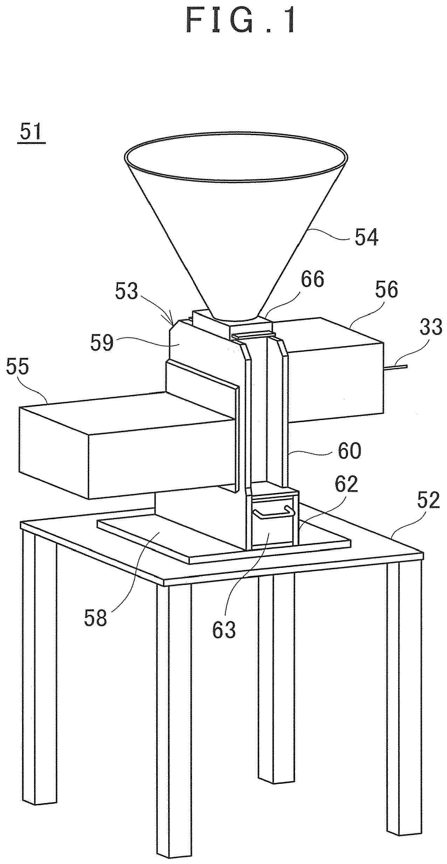

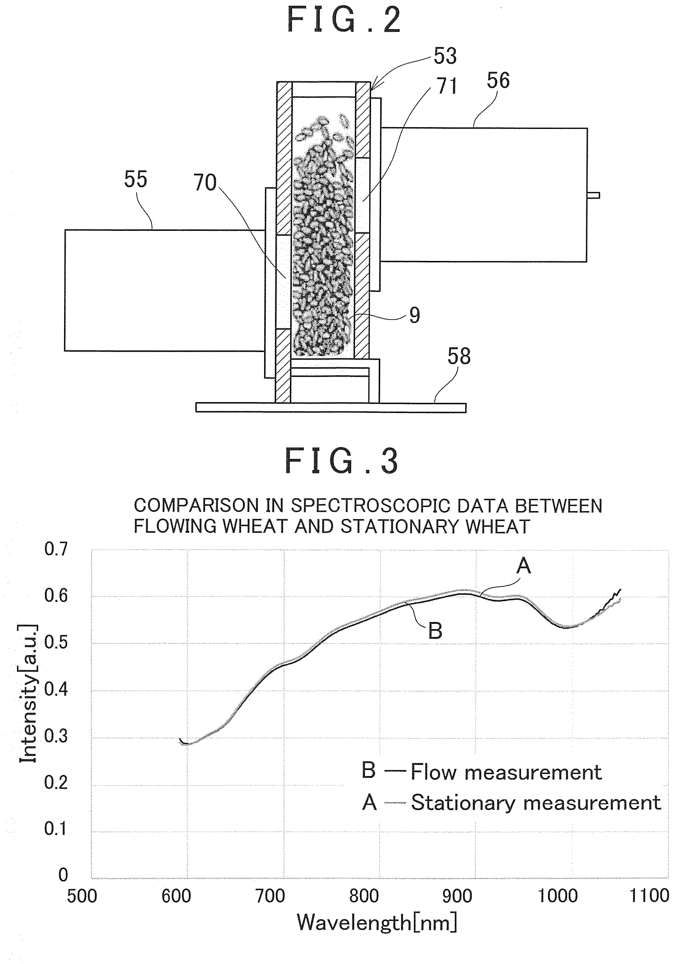

FIG.1 shows a schematic configuration of a granular-substance component measuring instrument 51 according to the present embodiment and shows a state in which the granular-substance component measuring instrument 51 is installed on a table 52. Further, FIG.2 shows a state in which a sample 9 as an object to be measured is filled in a sample holding device 53 (to be described later).

It is to be noted that, support means on which the granular-substance component measuring instrument 51 is installed is not limited to the table 52 but may be any one that can stably support the granular-substance component measuring instrument 51.

The granular-substance component measuring instrument 51 comprises the sample holding device 53 for mainly storing a sample, a sample conduct cylinder 54 for guiding a sample to the sample holding device 53, a first component analyzing instrument 55 mounted on one side wall of the sample holding device 53, and a second component analyzing instrument 56 mounted on a side wall opposite to the one side wall.

In each side wall of the sample holding device 53, a detection window (to be described later) is provided, and the first component analyzing instrument 55 and the second component analyzing instrument 56 are respectively configured to irradiate the sample with a detection light through a detection window, and to receive a reflected light from the sample through the detection window.

The first component analyzing instrument 55 and the second component analyzing instrument 56 may be spectroscopic analyzing instruments of a same type or spectroscopic analyzing instruments of different types. Further, in order to prevent a mutual interference of detection lights, optical axes of the first component analyzing instrument 55 and the second component analyzing instrument 56 are shifted. In the present embodiment, the optical axes are shifted in up and down direction. It is to be noted that, in an illustration, the first component analyzing instrument 55 and the second component analyzing instrument 56 are provided on different side walls so as to oppose, but the first component analyzing instrument 55 and the second component analyzing instrument 56 may be disposed up and down on the same side wall.

The sample holding device 53 comprises a displacement giving means (to be described later) which gives an intermittent or continuous displacement to a stored granular substance.

As the displacement giving means, it may be a vibrator which gives a vibration to a granular substance, an agitator which agitates a granular substance by rotating or reciprocating an agitating rod, a means for intermittently discharging a predetermined amount of a granular substance, or a means for realizing a flow state in a granular substance inside the sample holding device 53. It is to be noted that, in a case of realizing a flow state, an air may be discharged upward from a bottom part of the sample holding device 53 such that a granular substance is floated/moved or agitated. Alternatively, while supplying a sample from the sample conduct cylinder 54 to the sample holding device 53, the sample may be discharged from a bottom part of the sample holding device 53.

As a basic measuring method of the present embodiment, a displacement is given to a granular substance at each measurement, and a granular substance is measured in different states at each measurement.

As a measuring method by giving a displacement to a granular substance, a displacement is intermittently given to a granular substance, and a measurement may be performed in a state where a granular substance is stationary, or a measurement may be performed in a state where a displacement is dynamically given.

It is to be noted that, as a case while a displacement being given dynamically, it is possible to consider such a state where a vibration is applied to a granular substance or a granular substance is fluidized and the like.

First, the inventor performs a spectroscopic analysis in a state where a granular substance is stationary and in a state where a dynamic displacement is given to a granular substance, respectively, and measures an influence which a dynamic displacement exerts on an analysis result.

FIG.3 shows a result that a granular substance is wheat and a spectroscopic analysis is performed for wheat in a stationary state and in a flow state, respectively. In FIG.3, a horizontal axis indicates a wavelength, a vertical axis indicates a received light intensity, and a curve A indicates a measurement of a stationary state, and a curve B indicates a measurement in a flow state. Further, in a flow state, a moving speed of wheat is assumed to be approximately 0.05 m/second.

As shown in FIG.3, there is few difference between an analysis result of a stationary state and an analysis result of a flow state, and it is confirmed that a flow of wheat does not influence an analysis result. An analysis result in FIG.3 is with respect to wheat, but it is possible to acquire similar results even for other granular substances such as rice or soy beans, for instance.

Therefore, in the present embodiment, a measurement is performed in a state where a granular substance to be measured is dynamically displaced. By performing a plurality of times of measurements in a dynamically displaced state, measurement results are averaged, and a measurement accuracy is improved. It is to be noted that, in the embodiment as described above, two component analysis instruments, that is, the first component analyzing instrument 55 and the second component analyzing instrument 56 are provided on the sample holding device 53, but one component analyzing instrument may be provided.

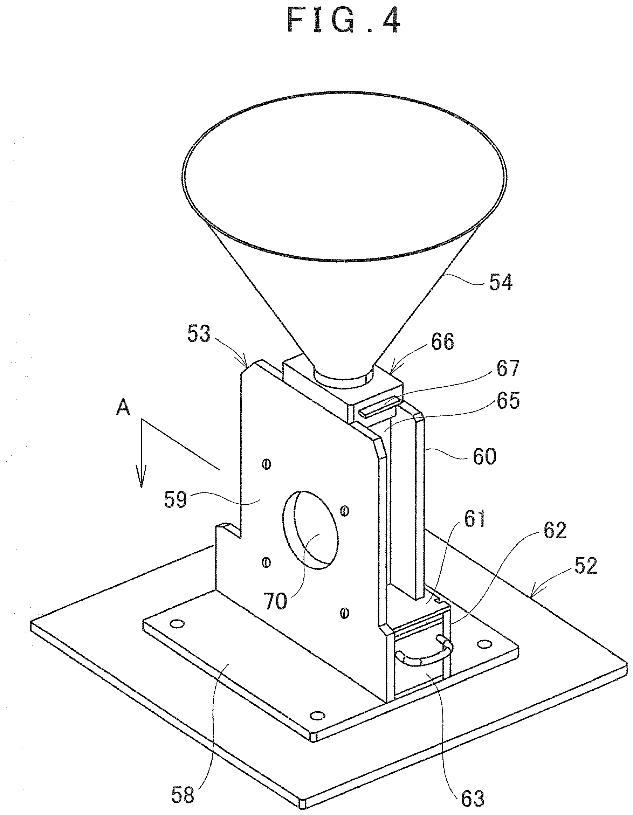

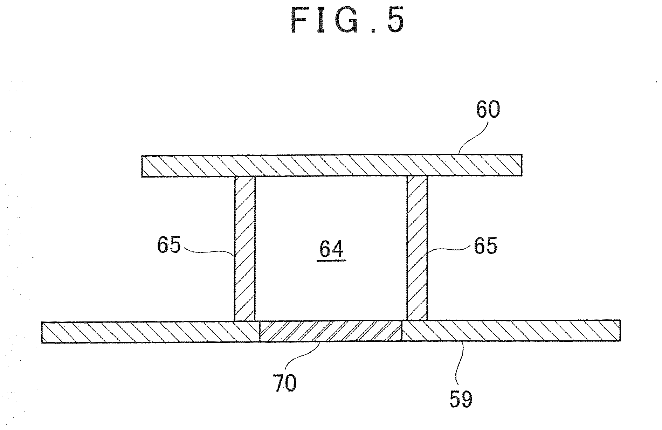

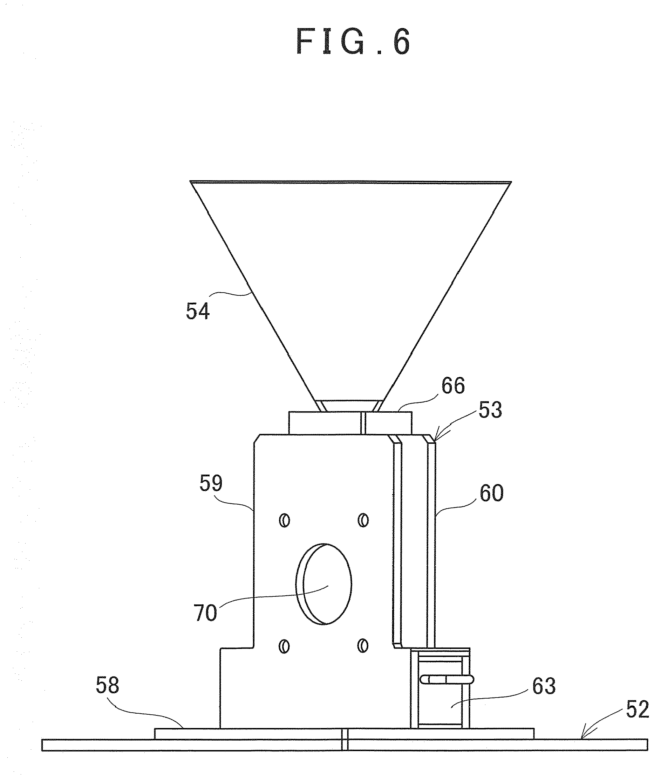

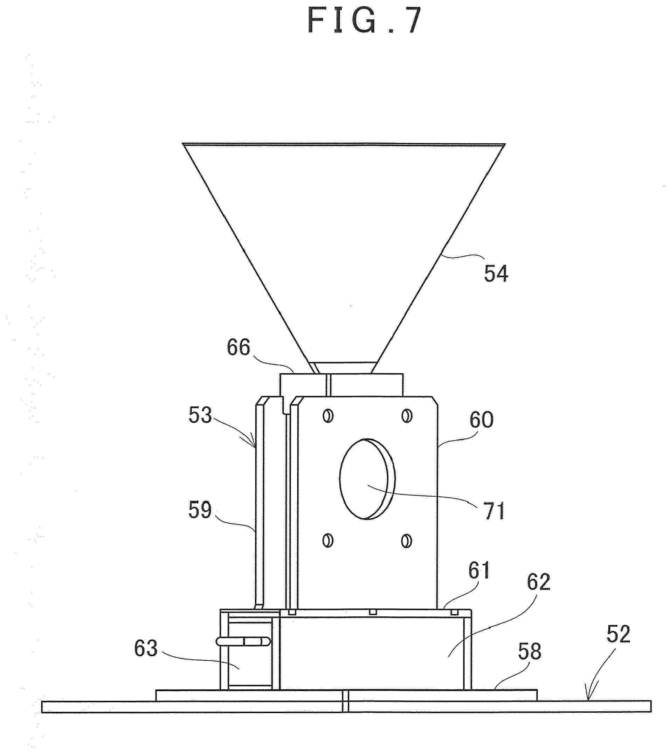

A description will be given on the sample holding device 53 by referring to FIG.1, FIG.2, FIG.4 to FIG.7.

The sample holding device 53 has a bottom board 58 on a lower end. On the bottom board 58, a first vertical board 59 is provided perpendicularly. A shelf board 61 in parallel to the bottom board 58 is provided on the first vertical board 59, and with the shelf board 61 as a top plate, a space surrounded by the shelf board 61, a lower part of the first vertical board 59, and a lateral plate 62 is formed on a lower side of the shelf board 61, and in the space, a drawer 63 is provided capable of putting-in/drawing-out.

A second vertical board 60 in parallel to the first vertical board 59 is provided perpendicularly on the shelf board 61. The first vertical board 59 and the second vertical board 60 oppose each other at a predetermined interval, and this interval is a known value.

Side plates 65, 65 which connect the first vertical board 59 and the second vertical board 60 are provided, and by the first vertical board 59, the second vertical board 60 and the side plates 65, 65, a columnar space 64 having a rectangular section and extending vertically is formed above the shelf board 61.

A discharge port (not shown), which communicates with the space 64, is formed in the shelf board 61, and the discharge port is capable of being opened/closed by a lower-part opening/closing valve (not shown). The lower-part opening/closing valve may be configured to be manually opened/closed or to be opened/closed by an actuator such as a solenoid. Further, in a case of being manually opened/closed, a function as an opening/closing valve may be given to the drawer 63. For instance, a valve hole is formed in an upper lid, a bottom plate of the drawer 63, at least in an upper lid in such a manner that the valve hole and the space 64 intermittently match by pulling-out/pushing-in of the drawer.

On an upper end of the space 64, a valve unit 66 is provided. A lower end of the sample conduct cylinder 54 is fixed to the valve unit 66, and the sample conduct cylinder 54 is provided on an upper end of the space 64 via the valve unit 66.

The sample conduct cylinder 54 has a funnel shape (that is, an inverted cone shape). It is to be noted that the shape of the sample conduct cylinder 54 is not limited to a funnel shape but only needs to be a shape that smoothly guides a sample into the space 64.

The valve unit 66 has an upper-part opening/closing valve 67, and an opening on a lower end of the valve unit 66 is opened/closed by the upper-part opening/closing valve 67. Then, in an open state of the upper-part opening/closing valve 67, the sample conduct cylinder 54 communicates with the space 64, and the space 64 is brought into a sealed state on the condition that the upper-part opening/closing valve 67 is closed and the lower-part opening/closing valve is closed.

The upper-part opening/closing valve 67 may be opened/closed manually, or the valve unit 66 may have an actuator such as a solenoid, and the upper-part opening/closing valve 67 may be opened/closed by the actuator. In the present embodiment, the lower-part opening/closing valve and the upper-part opening/closing valve 67 are assumed to be opened/closed by an actuator.

In the embodiment as described above, when the upper-part opening/closing valve 67 is open, the lower-part opening/closing valve is closed, and a sample 9 is input into the sample conduct cylinder 54, the space 64 is filled with the sample 9. After the filling, the upper-part opening/closing valve 67 is closed.

When the sample 9 is filled in the space 64, it becomes possible to perform a measurement. By opening/closing intermittently a lower-part opening/closing valve in a measurement state, the sample 9 in the space 64 is intermittently discharged, and a movement of the sample 9 occurs in the space 64. That is, a displacement is given to the sample 9 (granular substance). Therefore, the lower-part opening/closing valve functions as a displacement giving means.

Further, by adjusting an opening degree of the lower-part opening/closing valve in such a manner that the sample 9 (granular substance) is continuously discharged in a predetermined amount, it is possible to bring the sample 9 in the space 64 into a flow state. In this case, the lower-part opening/closing valve also functions as a displacement giving means. Further, making the upper-part opening/closing valve 67 open, the sample 9 may be continuously supplied.

Further, a plurality of air ejection ports are provided in the shelf board 61, an air is ejected from the air ejection port to the space 64, and the sample 9 may be agitated or floated/moved by the ejected air. In this case, the air ejection port and an ejected air function as a displacement giving means. In addition, as the displacement giving means, a vibrator, an agitator, and the like can be considered as described above.

The sample 9 having been measured is discharged to the drawer 63, and taking-out of the sample 9 from the sample holding device 53 is performed by pulling out the drawer 63.

Therefore, in the case of supplying the sample 9, it is only necessary to input the sample 9 to the sample conduct cylinder 54, and in the case of taking-out, it is possible to take the sample 9 out by the drawer 63. Therefore, it is possible to make a replacement of the sample 9 easily.

It is to be noted that, a large-sized container is provided below the table 52, and a measured sample may be directly discharged into the container.

On a part facing the space 64 of the first vertical board 59, a first detection window 70 is provided, and the first detection window 70 is closed airtightly by a transparent member such as a glass. Further, on a part facing the space 64 of the second vertical board 60, a second detection window 71 is provided, and the second detection window 71 is closed airtightly by a transparent member such as a glass. A transparent member such as a glass may be provided on a light receiving part of a sensor other than a detection window.

The first detection window 70 and the second detection window 71 are provided at positions shifted in up and down direction.

The first component analyzing instrument 55 is mounted on the first vertical board 59 such that a center of the first detection window 70 matches a detection-light emission optical axis of the first component analyzing instrument 55. The first component analyzing instrument 55 is capable of being attached/detached with respect to the first vertical board 59.

The second component analyzing instrument 56 is mounted on the second vertical board 60 in such a manner that a center of the second detection window 71 is coincident with a detection-light emission optical axis of the second component analyzing instrument 56. The second component analyzing instrument 56 is capable of being attached/detached with respect to the second vertical board 60.

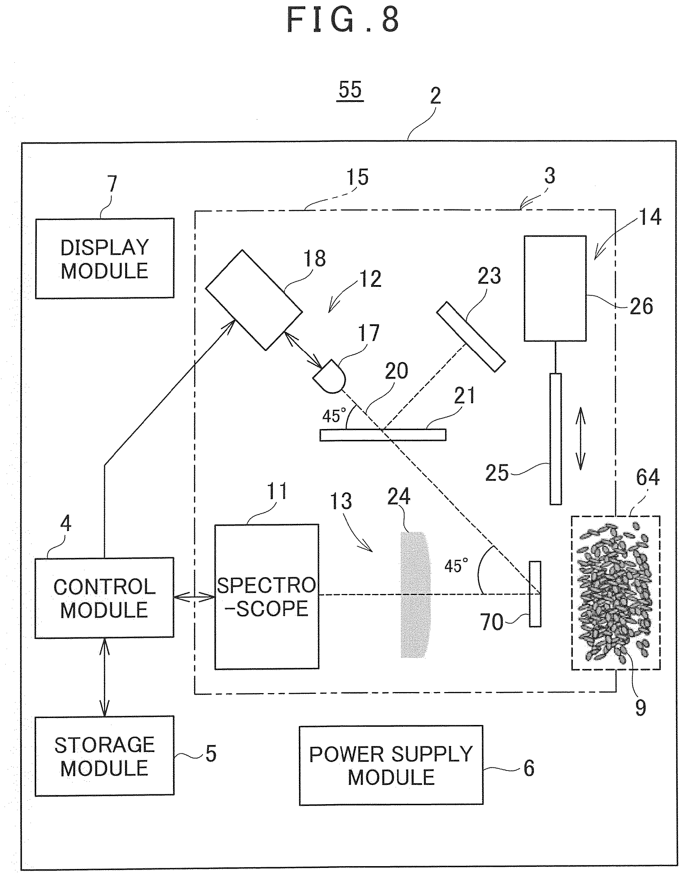

First, a description will be given on the first component analyzing instrument 55 using a white LED as a light emission source by referring to FIG.8.

In FIG.8, a reference numeral 2 denotes a casing, and a component detection sensor 3, a control module 4, a storage module 5, a power supply module 6, a display module 7 and the like are provided inside the casing 2. The power supply module 6 supplies a power to the component detection sensor 3, the control module 4, the display module 7 and the like. Further, in FIG.8, a reference numeral 9 denotes a sample filled in the space 64.

In the storage module 5, various types of programs for the first component analyzing instrument 55 to perform spectroscopic measurements and a program for the first component analyzing instrument 55 to calculate content rates of a water, a protein and the like based on a spectroscopy data acquired by spectroscopic measurements are stored. Further, in the storage module 5, a spectroscopy data acquired by spectroscopic measurements, and a calculation result of content rates and the like are stored.

The control module 4 executes a program stored in the storage module 5 and executes required controls at required timings with respect to a spectroscope 11 (to be described later), a calibration module 14 (to be described later), a driver 18 (to be described later) and the like. Further, the control module 4 executes controls also with respect to an opening/closing of a lower-part opening/closing valve and an opening/closing of the upper-part opening/closing valve 67.

It is to be noted that, as the storage module 5, a semiconductor memory such as a RAM, a ROM, a FlashROM, a DRAM and the like, a magnetic recording memory such as a HDD and the like are used. Further, as the control module 4, a CPU specialized to the present embodiment or a general-purpose CPU, an embedded CPU, a microprocessor and the like are used.

Subsequently, a description will be given on the component detection sensor 3.

The component detection sensor 3 mainly includes the spectroscope 11, a light source module 12, a photodetector 13, the calibration module 14. The spectroscope 11, the light source module 12, the photodetector 13 and the calibration module 14 may be accommodated in a sensor case 15, and the component detection sensor 3 may be unitized.

Electrical powers are supplied from the power supply module 6 to the spectroscope 11, the light source module 12, the photodetector 13 and the calibration module 14, respectively.

The spectroscope 11 has an energy-dispersive Si detector for which an Si single crystal is used as a detection element and is configured such that the detection element spectro-analyzes a received light. Further, a range capable of analyzing by an Si detector is a near-infrared light with a wavelength of 1100 nm or less.

The light source module 12 has a white LED 17 which emits a white light as a light emission source and the driver 18 which makes the white LED 17 emit light. An electrical power is supplied to the driver 18 from the power supply module 6, and a light emission is controlled by the control module 4. It is to be noted that a case in which a light emission source and the driver 18 are integrally constituted is shown, but the light emission source and the driver 18 may have a separate constitution.

An optical filter 21 is disposed on an optical path (optical axis) of the white LED 17. As described later, the optical filter 21 has an optical characteristic which shuts off or dims a light in a visible light region and transmits a wavelength band required for a component detection.

For the white LED 17, it is possible to use a white LED in a market, a light emitting element and a lens are integrated, and a light beam from the light emission element is set to be a parallel light or to have a predetermined spread angle. Further, in the present embodiment, since it is possible to shorten a light projection distance to the sample 9, it is possible to directly irradiate the sample 9 with a light emitted from the white LED 17 without going through an optical system such as a lens. Therefore, in the present embodiment, it is possible to omit a light-projecting optical system.

Subsequently, in the present embodiment, a water molecule and molecules constituting a protein, as contained in the sample 9, are detected by a spectroscopic analysis.

A water shows a relatively strong absorption at 1940 nm, 1450 nm, 1190 nm, 970 nm, and 760 nm. Further, a protein shows a complicated spectrum in which absorptions by various functional groups are overlapped, but it is known to have a characteristic absorption band at 2180 nm, 2050 nm.

Therefore, it is preferable to use a long wavelength of 1000 nm or more for a near-infrared spectroscopy, but an expensive InGaAs detector should be used for this wavelength band. Thus, in the present embodiment, a near-infrared spectroscopy is performed by using a wavelength range of a wavelength 1100 nm or less, and it is possible to use a Si detector.

A white light is emitted from the white LED 17, and the white light contains substantially the entire region of wavelength bands. It is preferred to shut off the light other than wavelength band required for detecting component. Further, as a characteristic of a white LED, since output of longer wavelength region than infrared region (900 nm) is drastically smaller than output of visible region (1/10, for instance), a saturation of a spectral sensitivity in a visible light region should be avoided.

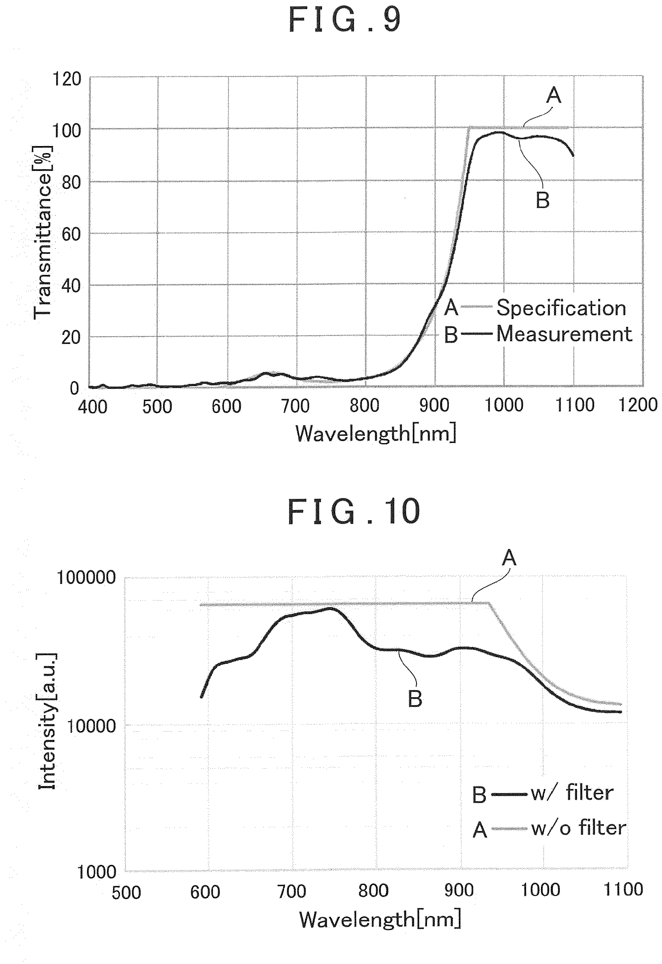

FIG.9 is a graph showing an optical characteristic of the optical filter 21, and a vertical axis indicates a transmittance and a horizontal axis indicates a wavelength.

Further, in FIG.9, a curve A is a wavelength/transmittance curve required in the present embodiment, and a curve B is an actual wavelength/transmittance curve of the optical filter 21 used in the present embodiment.

The curve A and the curve B substantially match, and the optical filter 21 has a transmittance of substantially 100% in a wavelength band from 950 nm to 1100 mm as used in the present embodiment.

That is, the optical filter 21 has an optical characteristic which transmits substantially 100% of a wavelength range of a wavelength from 1100 nm to 950 nm and cuts off a light in a wavelength region (visible light region) at 950 nm or less to approximately 0%.

In the present embodiment, an optical characteristic of the optical filter 21 is set to 2.5% transmittance with respect to a visible region and substantially 100% transmittance with respect to an infrared region. By allowing a transmission of 2.5% also with respect to a light in a visible region, as described later, it becomes possible to spectro-analyze molecules other than water molecules, protein molecules.

It is to be noted that, it is considered that an optimal transmittance of the optical filter 21 is different depending on the white LED 17 to be used and is not limited to a transmittance described above.

Therefore, a light irradiated the sample 9 has a wavelength range of a wavelength from 1100 nm to 950 nm. Since the irradiated light is limited to a wavelength band required for a component detection, an S/N of a detection result is improved. Further, it is also possible to avoid a saturation of a spectral sensitivity in a visible light region.

A light 20 emitted from the white LED 17 is transmitted through the optical filter 21 and is irradiated to the sample 9 through the first detection window 70.

The optical filter 21 and the first detection window 70 are inclined by a predetermined angle with respect to an optical axis of the white LED 17. Thereby, a light reflected by the optical filter 21 and the first detection window 70 do not enter the white LED 17, and a light emission state in the white LED 17 is prevented from becoming unstable. It is to be noted that, in an illustration, the optical filter 21 and the first detection window 70 are inclined by 45° with respect to an optical axis, respectively, but the inclined angle is not limited to this angle.

The optical filter 21 transmits a wavelength of 1100 nm or less, but at the same time, reflects a white light at a surface of the optical filter 21. In the present embodiment, a light absorbing plate 23 is provided on a reflection optical axis of the white light. The light absorbing plate 23 absorbs a reflected light from the optical filter 21. The light absorbing plate 23 prevents a light reflected by the optical filter 21 from irregularity reflecting inside the casing 2 and prevents a detection result by the spectroscope 11 from being influenced thereby.

A diffused reflected light reflected by the sample 9 and transmitted through the first detection window 70 is condensed by a condenser lens 24 and is incident to the spectroscope 11.

The spectroscope 11 spectro-analyzes a reflected light being incident and outputs a spectroscopy data to the control module 4.

The control module 4 acquires a reflected diffused spectrum based on a spectroscopy data, further calculates contents of a moisture and a protein and displays calculation results of a reflected diffused spectrum, a spectroscopy data, a protein content rate, a moisture content rate, and the like on the display module 7.

Next, in the present embodiment, in order to guarantee a measurement accuracy, the calibration module 14 is provided.

The calibration module 14 has at least a standard white reflective board 25 and a white-board insertion/removal module 26.

The white-board insertion/removal module 26 inserts/removes the standard white reflective board 25 into/from an optical path of the light emitted from the white LED 17. Before and after a component analysis of the sample 9 or either one of before and after, or every predetermined time, the standard white reflective board 25 is inserted into an optical path between the optical filter 21 and the space 64, the light 20 is irradiated on the standard white reflective board 25, and a reflected light from the standard white reflective board 25 is detected by the spectroscope 11 as a reference light for a calibration. Further, a driving of the white-board insertion/removal module 26 and a driving timing are controlled by the control module 4.

Next, a description will be given on a relationship between the optical filter 21 and the standard white reflective board 25.

As described above, in the present embodiment, a light beam in a wavelength region from 950 nm to 1100 nm (particularly, from 1000 nm to 1100 nm) is required, and when the white LED 17 is caused to emit a light such that a light intensity with a wavelength of this band is suitable for a measurement, a light intensity with a wavelength in a visible region (a wavelength at 950 nm or less) becomes strong, and a photodetector of the spectroscope 11 is saturated.

FIG.10 shows a reflected spectroscopy data of the standard white reflective board 25 acquired by inserting the standard white reflective board 25 into an optical path. In FIG.10, a vertical axis indicates an intensity of a light receiving signal, and a horizontal axis indicates a wavelength. Further, in the drawing, a curve A is a reflective spectroscopy data in a state without the optical filter 21, and a photodetector is saturated at a wavelength of 950 nm or less.

Next, a curve B shows a reflective spectroscopy data of the standard white reflective board 25 when the light 20 is emitted through the optical filter 21. The curve B shows that, by providing the optical filter 21, it is possible to acquire a reflective spectroscopy data without a saturation in all wavelength regions.

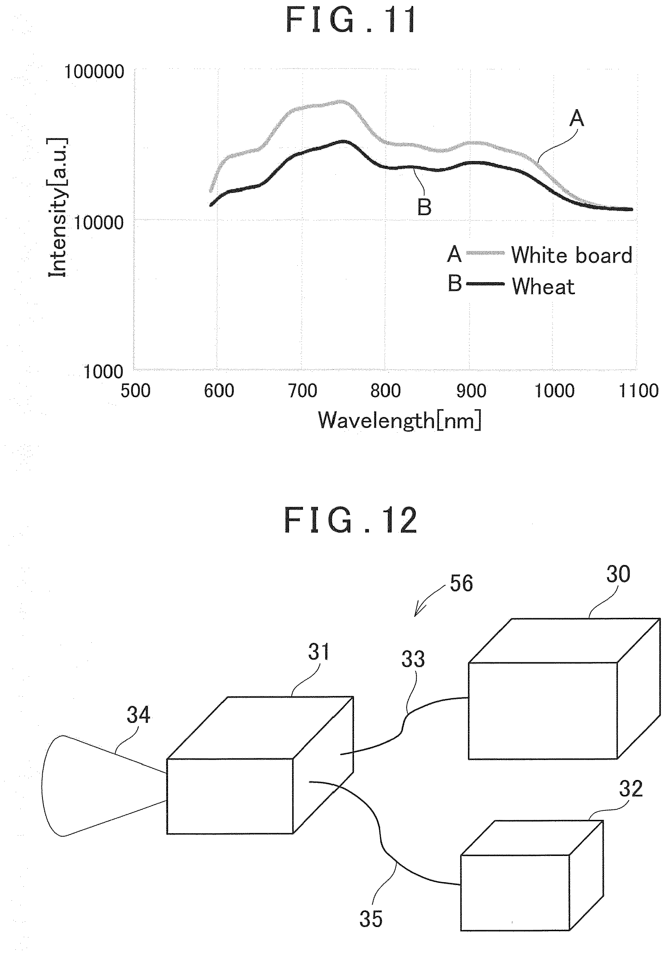

FIG.11 shows a reflective spectroscopy data of a standard white reflective board as a curve A and a reflective spectroscopy data from the sample 9 (wheat in the present embodiment) as a curve B, in a state where the optical filter 21 is provided. In FIG.11, a vertical axis indicates an intensity of a light receiving signal, and a horizontal axis indicates a wavelength.

A curve A shows a reflective spectroscopy data in a calibration state.

A curve B shows that it is possible to also acquire a light intensity required for a measurement with respect to a reflected light from a sample in all wavelength regions, and an effectiveness of the optical filter 21 is shown. Therefore, by a combination of the white LED 17 and the optical filter 21, it is possible to also perform a component analysis with respect to an object to be measured other than grain.

By performing a calibration, it is possible to detect a light emission state of the light source module 12 and abnormality of a measurement state, and the like.

Next, a description will be given on a second component analyzing instrument 56 using a halogen lamp as a light emission source by referring to FIG.12.

FIG.12 shows general features of the second component analyzing instrument 56, and the second component analyzing instrument 56 using a halogen lamp as shown in FIG.12, a light source module 30, a measurement optical system 31 and a spectroscope 32 are configured separately and independently, and the measurement optical system 31 is mounted on the second vertical board 60 as a second component analyzing instrument 56 in FIG.1. The light source module 30 and the measurement optical system 31 are connected by an optical fiber 33, and the measurement optical system 31 and the spectroscope 32 are connected by an optical fiber 35.

A light emitted by the light source module 30 (halogen lamp) is led to the measurement optical system 31 by the optical fiber 33 and is irradiated to an object (not shown) as a detection light 34 from the measurement optical system 31. The detection light 34 reflected by an object (reflected detection light 34') is received by the measurement optical system 31 and enters the spectroscope 32 through an optical fiber 35. In the spectroscope 32, the reflected detection light 34' is optically analyzed, a light absorbing amount in a specified wavelength band is detected, and protein/moisture content rates of grain are measured.

A halogen lamp used in the grain-component analyzing instrument described above has a high heat generation amount, a high power consumption and thus, each unit is connected to each other by the optical fibers 33, 35 such that a heat generation does not influence a measurement.

In the present embodiment, a setting of a sample can be performed only by inputting a sample in the sample conduct cylinder 54, the setting of the sample is easy. Further, a taking-out of a sample can be also performed only by pulling-out by the drawer 63, and a replacement of a sample is also easy.

An analysis result measured by the first component analyzing instrument 55 and an analysis result measured by the second component analyzing instrument 56 may be input in the control module 4 of the first component analyzing instrument 55 or a control module (not shown) of the second component analyzing instrument 56 and may be averaged. Alternatively, the analysis result measured by the first component analyzing instrument 55 and the analysis result measured by the second component analyzing instrument 56 may be input in a terminal, which can be connected to the first component analyzing instrument 55 and the second component analyzing instrument 56, and may be averaged.

In the present embodiment, since it is possible to measure the same sample at the same time by a plurality of component analyzing instruments, by averaging measurement results of a plurality of component analyzing instruments, measurement results, in which influences of individual differences of component analyzing instruments are offset, are acquired. Further, one of a plurality of component analyzing instruments is set as a reference instrument, and it becomes possible to calibrate measurement results of the other component analyzing instrument based on a measurement result of the reference instrument. Further, a component analyzing instrument of a different type is provided, measurement result of each model may be compared each other, and the other measurement result may be corrected by one measurement result, or measurement results of the both may be averaged.

Further, in the present embodiment, since measurements are performed a plurality of times while displacing (moving) granular substances (objects), a variation in measurement results caused by a difference in states of objects is offset, and a measurement result with a high accuracy is acquired.

Further, in the present embodiment, since it is possible to measure an object in a flow state, a part of grain while harvesting is made to flow to the sample holding device 53, and thereby it is possible to perform a measurement in real time. Therefore, it is possible to measure a quality of grain while harvesting by mounting a granular-substance component measuring instrument according to the present embodiment on an agricultural machine or the like.

## Claims

1. A granular-substance component measuring method comprising: steps of emitting detection light to granular substances filled in a space (64), receiving a reflected light from said granular substances and measuring a component of granular substances by a spectroscopy, wherein a displacement is given to said granular substances at each measurement, measurements are performed a plurality of times, and measurement results are averaged.

2. The granular-substance component measuring method according to claim 1, wherein a displacement is intermittently given to said granular substances, and a measurement is performed while said granular substances are in a stationary state.

3. The granular-substance component measuring method according to claim 1 or 2, wherein said granular substances are fluidized or agitated, and a measurement is performed in a state where a dynamic displacement is given to said granular substances.

4. A granular-substance component measuring instrument comprising: a sample holding device (53) with a space (64) in which granular substances are filled and at least one component analyzing instrument (55) mounted on said sample holding device, wherein a detection window (70) is provided in a board facing said space (64), wherein said component analyzing instrument is configured to irradiate a detection light to said granular substances through said detection window, to receive a reflected light from said granular substances, and to measure components by a spectroscopic analysis, wherein said sample holding device has a displacement giving means for giving a displacement to said granular substances, and is configured to give a displacement said granular substances by said displacement giving means, and wherein said component analyzing instrument is configured to perform measuring granular substances a plurality of times wherein a displacement is given to said granular substances at each measurement and to average measurement results.

5. The granular-substance component measuring instrument according to claim 4, wherein detection windows (70, 71) are provided in two opposing boards facing said space (64) respectively, component analyzing instruments (55, 56) are provided at said detection windows respectively, and components of said granular substances are measured through said detection windows by respective said component analyzing instruments.

6. The granular-substance component measuring instrument according to claim 5, wherein one of said component analyzing instruments (55, 56) is a reference-component analyzing instrument, and a measurement result of the other component analyzing instrument is calibrated by a measurement result of said reference-component analyzing instrument.

7. The granular-substance component measuring instrument according to claim 5 or 6, wherein said two component analyzing instruments (55, 56) are component analyzing instruments of a same type or different types.

8. The granular-substance component measuring instrument according to one of claims 5-7, wherein an upper-part opening/closing valve (67) is provided on an upper end of said space (64), a lower-part opening/closing valve is provided on a lower end of said space, and a displacement is given to granular substances inside said space at least by an opening/closing of a lower-part opening/closing valve.

9. The granular-substance component measuring instrument according to claim 8, wherein at least said lower-part opening/closing valve is opened by a predetermined opening degree, said granular substances are discharged, and granular substances in said space (64) are adapted to flow.

10. The granular-substance component measuring instrument according to claim 5, wherein an air is ejected from a lower end part of said space (64), and the granular substances are adapted to float/move or to be agitated.
